# EUROPÄISCHE PATENTANMELDUNG

(11) **EP 1 946 699 A2**
(43) Veröffentlichungstag der Anmeldung: **23.07.2008**
(21) Anmeldenummer: 08000442.7
(22) Anmeldetag: 11.01.2008
(51) Int. Cl.: A61B 5/0408, A61B 7/04

(54) **Gerät zur Aufnahme kardiologischer Untersuchungsdaten an einem Patienten**

(30) Priorität: 20.01.2007 DE 102007003021
(71) Anmelder: Heat GmbH, 72764 Reutlingen (DE)
(72) Erfinder: Schlüren, Markus, 72764 Reutlingen (DE)
(74) Vertreter: Kohler Schmid Möbus

(57) **Zusammenfassung**

Ein Gerät (1) zur Aufnahme kardiologischer Untersuchungsdaten an einem Patienten (2) weist mehrere Elektroden (3a, 3b, ...) und mehrere in die Elektroden integrierte und oder an diesen befestigte Schallaufnahmevorrichtungen auf, die am Körper des Patienten (2) befestigbar und mit einer Signalaufnahmeeinheit (5) verbunden sind. Mit Hilfe des Geräts (1) werden zeitgleich elektrische und akustische Signale vom Körper des Patienten (2) erfasst. Die gemessenen Untersuchungsdaten können sowohl mit Hilfe einer Anzeigevorrichtung (6) als Elektro- bzw. Phonokardiogramm dargestellt als auch in einer Datenverarbeitungsanlage (12) zusätzlich gemeinsam ausgewertet und als Interpretation visuell angezeigt oder ausgedruckt werden.

## Beschreibung

Die Erfindung betrifft ein Gerät zur Aufnahme kardiologischer Untersuchungsdaten an einem Patienten, umfassend mehrere am Körper des Patienten befestigbare Elektroden und eine Signalaufnahmeeinheit, die mit den einzelnen Elektroden verbunden ist und die von den Elektroden am Körper des Patienten erfassten Signale als Untersuchungsdaten aufnimmt.

Ein derartiges Gerät, das beispielsweise in der DE 299 177 80 U1 gezeigt und beschrieben ist, wird in der Medizin zur Durchführung elektrokardiographischer Untersuchungen eingesetzt. Die elektrische Aktivität des Herzens bzw. des Herzmuskels wird mit Hilfe der Elektroden an der Hautoberfläche des Patienten abgeleitet und gemessen. Bei der Aufzeichnung der Messwerte über die Zeitachse entsteht ein immer wiederkehrendes Bild der elektrischen Herzaktivität. Dieses Bild, ein Elektrokardiogramm oder eine Herzstromkurve, gibt Auskunft über Herzfunktionen, wie Herzrhythmus und -frequenz, sowie über etwaige Störungen derselben. Eine kardiologische Basisuntersuchung umfasst das Aufzeichnen eines Elektrokardiogramms, d.h., die Durchführung einer Elektrokardiographie, ein Messen des Blutdrucks des Patienten und eine Auskultation seines Herzens. Bei der Herzauskultation werden die Schallphänomene des Herzens, Herztöne und -geräusche, durch den Arzt unter Zuhilfenahme eines Stethoskops abgehört und analysiert. Die Qualität der Auskultations-Befunde hängt wesentlich vom Gehör des untersuchenden Arztes sowie seiner kardiologischen Fachkenntnisse und Erfahrung ab. Die Herzauskultation kann auch apparativ über eine Phonokardiographie erfolgen, wobei der Herzschall mittels eines Mikrophons und eines Verstärkers aufgezeichnet wird.

Der Erfindung liegt die Aufgabe zugrunde, eine Möglichkeit zu schaffen, eine kardiologische Basisuntersuchung in vereinfachter Weise und bei einem geringeren zeitlichen Aufwand durchzuführen und dabei Untersuchungsdaten für eine verbesserte Diagnostik bereitzustellen.

Gelöst wird die Aufgabe durch ein Gerät der eingangs beschriebenen Art, das erfindungsgemäß dadurch gekennzeichnet ist, dass das Gerät zumindest eine mit der Signalaufnahmeeinheit verbundene, an den Elektroden befestigbare oder in diese integrierte Schallaufnahmevorrichtung aufweist.

Mit Hilfe der Schallaufnahmevorrichtung, wie einem Mikrophon, können Schallphänomene des Herzens aufgenommen werden. Die Schallaufnahmevorrichtung kann auch als eine auf einen Brustkorb aufsetzbare Kalotte oder als ein Stempel zur Detektion von Schwingungen der Haut ausgebildet sein. Das erfindungsgemäße Gerät erlaubt eine gleichzeitige Aufnahme von elektrischen Signalen über die Elektroden sowie von akustischen Signalen über die Schallaufnahmevorrichtung. Die in der Signalaufnahmeeinheit aufgenommenen Untersuchungsdaten können sowohl für eine Elektrokardiographie als auch für eine Phonokardiographie verwendet werden. Durch die simultane Aufnahme von Untersuchungsdaten ist der zeitliche Aufwand für die Datenaufnahme und folglich für die gesamte kardiologische Basisuntersuchung verkürzt.

Die Elektroden, in welche eine oder mehrere Schallaufnahmevorrichtungen integriert sind, sind typischerweise als Klebe- oder Saugelektroden ausgebildet und somit in einfacher Weise am Körper des Patienten zu befestigen.

In einer weiteren bevorzugten Ausführungsform des erfindungsgemäßen Geräts weist zumindest eine Schallaufnahmevorrichtung Mittel zum Anschluss an eine Elektrode und/oder an die Signalaufnahmeeinheit, insbesondere einen Anschlussadapter, auf. Diese Ausführungsform bietet den Vorteil, dass die Elektroden abhängig von den gewählten Befestigungspunkten am Körper des Patienten, mit anderen Worten abhängig von der gewählten Ableitungsmethode, bedarfsgerecht mit einer oder mehreren Schallaufnahmevorrichtungen bestückt werden können.

Das erfindungsgemäße Gerät weist vorteilhafterweise eine visuelle und/oder akustische Anzeigevorrichtung zur Anzeige erfasster Untersuchungsdaten auf. Die mit Hilfe des erfindungsgemäßen Geräts ermittelten Untersuchungsdaten sind unmittelbar sicht- bzw. hörbar, was eine Kurzdiagnose und insbesondere die sofortige Anzeige auffälliger Anomalien ermöglicht.

Das Gerät weist zweckmäßigerweise eine Schnittstelle zur Ausbildung einer Datenverbindung mit einer Datenverarbeitungsanlage auf. Die mit Hilfe des erfindungsgemäßen Geräts aufgenommenen Untersuchungsdaten können auf die Datenverarbeitungsanlage übertragen und dort ausgewertet, insbesondere einer verbesserten Diagnostik zugeführt werden.

Vorteilhafterweise weist das Gerät einen Datenspeicher zum Speichern der erfassten Untersuchungsdaten auf. Dies erlaubt einen Betrieb des Geräts unabhängig von einer Datenverarbeitungsanlage. Das erfindungsgemäße Gerät kann zusätzlich eine eigene Stromversorgung, wie eine Batterie, aufweisen und somit eine unabhängige Einheit zur Abnahme kardiologischer Untersuchungsdaten an einem Patienten darstellen. Dies erlaubt den Einsatz des erfindungsgemäßen Geräts außerhalb von stationären Untersuchungseinrichtungen, wie Kliniken oder Arztpraxen, beispielsweise bei Hausbesuchen oder im rettungsmedizinischen Bereich.

Der Einsatz des erfindungsgemäßen Geräts beschränkt sich nicht auf die Herzmedizin, sondern umfasst sämtliche medizinische Bereiche, wie beispielsweise die Allgemein-, Arbeits- oder Flugmedizin. Eine Verwendung in der Tiermedizin ist ebenso vorstellbar. Die Erfindung stellt eine Erweiterung der elektrokardiographischen Diagnostik mit Elementen der Phonokardiographie dar. Es ist darüber hinaus denkbar, eine Ultraschallvorrichtung zur Durchführung einer Echokardiographie an dem erfindungsgemäßen Gerät vorzusehen.

Das Gerät weist vorteilhafterweise eine Eingabevorrichtung zur Eingabe weiterer Untersuchungsdaten auf. Über die Eingabevorrichtung kann beispielsweise der Blutdruck des Patienten eingegeben und bei der Auswertung der Untersuchungsdaten berücksichtigt werden. Alle im Rahmen einer kardiologischen Basisuntersuchung erforderlichen Untersuchungsdaten liegen somit in dem erfindungsgemäßen Gerät vor und können einer gemeinsamen Auswertung zugeführt werden.

Die kardiographischen Untersuchungsdaten können mit Hilfe des erfindungsgemäßen Geräts an einem Patienten, beispielsweise bei einem Hausbesuch, abgenommen werden, woraufhin eine Kurzdiagnose mit Hilfe der angezeigten Signale bzw. Untersuchungsdaten erstellt werden kann. Zu einem späteren Zeitpunkt kann eine detailliertere Auswertung der Untersuchungsdaten an einer Datenverarbeitungsanlage stattfinden.

Das Gerät ist bevorzugterweise als Drei-, Sechs- oder Zwölfkanalgerät ausgebildet und kann somit für sämtliche bekannte Ableitungsmethoden eingesetzt werden.

Es versteht sich, dass auch alle gewünschten Auswertungen und/oder Vergleiche mit Referenzdaten im Gerät zur Aufnahme von kardiologischen Untersuchungsdaten erfolgen können.

Die Erfindung umfasst weiter ein Verfahren zur Durchführung einer kardiologischen Untersuchung an einem Patienten unter Verwendung des erfindungsgemäßen Geräts, wobei in einem ersten Verfahrensschritt die Elektroden und die mindestens eine Schallaufnahmevorrichtung am Körper des Patienten befestigt werden, in einem zweiten Verfahrensschritt elektrische und akustische Signale vom Körper des Patienten erfasst werden und in einem dritten Schritt die Elektroden und die zumindest eine Schallaufnahmevorrichtung vom Patienten abgenommen werden. In dem erfindungsgemäßen Verfahren werden gleichzeitig elektro- und phonokardiographische Untersuchungsdaten erfasst.

In einer bevorzugten Variante des erfindungsgemäßen Verfahrens wird in einem vierten Verfahrensschritt das Gerät an eine Datenverarbeitungsanlage angeschlossen und in einem fünften Verfahrensschritt werden die aufgenommenen Untersuchungsdaten durch die Datenverarbeitungsanlage ausgewertet. Der vierte Verfahrensschritt ist dabei alternativ vor und der fünfte Verfahrensschritt gleichzeitig mit dem zweiten Verfahrensschritt, d.h., der Aufnahme von Untersuchungsdaten, ausführbar. Der zuvor in die Datenverarbeitungsanlage und/oder in das Gerät eingegebene Blutdruck des Patienten, die Herzstromkurve bzw. das Elektrokardiogramm und das mit der zumindest einen Schallvorrichtung detektierte Herzschallbild bzw. Phonokardiogramm können gemeinsam ausgewertet werden. Dadurch kann einerseits eine durch kardiologisches Expertenwissen ergänzte Basisuntersuchung mit rechnergestützer Diagnostik erfolgen und andererseits die in herkömmlicher Weise für die Auskultation aufgewendete Zeit eingespart werden.

In einem weiteren erfindungsgemäßen Verfahren zum Betrieb des erfindungsgemäßen Geräts findet zunächst ein Anschluss des Geräts an eine Datenverarbeitungsanlage und anschließend eine Auswertung der Untersuchungsdaten durch die Datenverarbeitungsanlage statt. Dabei werden vorteilhafterweise vor Anschluss des Geräts an die Datenverarbeitungsanlage mithilfe des Geräts Untersuchungsdaten aufgenommen und/oder Untersuchungsdaten in das Gerät eingegeben.

In einer weiteren bevorzugten Variante der erfindungsgemäßen Verfahren werden die Untersuchungsdaten mit in der Datenverarbeitungsanlage gespeicherten Referenzdaten verglichen und ein Untersuchungsergebnis ausgegeben. Das beim Aufzeichnen der Herzkurve gleichzeitig festgestellte Herzgeräuschmuster, insbesondere der Ort der maximalen Lautstärke (punktum maximum), wird mit in der Datenverarbeitungsanlage gespeicherten Geräuschmustern von bekannten Auskultationsbefunden verglichen und auf diese Weise eine Verdachtsdiagnose erstellt. Das erfindungsgemäße Gerät bzw. die erfindungsgemäße Verfahren erlauben eine frühzeitige Aufdeckung von in herkömmlicher Weise kaum feststellbaren Fehlfunktionen, beispielsweise einer Herzklappenstörung.

Das Untersuchungsergebnis kann an einem Bildschirm angezeigt und/oder über eine an die Datenverarbeitungsanlage angeschlossene Druckereinheit ausgedruckt werden. Neben einem Untersuchungsergebnis können die Messwerte selbst, d.h., eine Herzstromkurve, ein Herzschallbild und gegebenenfalls weitere Parameter, wie der Blutdruck des Patienten, ausgegeben werden. Die Untersuchungsdaten können zudem in der Datenverarbeitungsanlage gespeichert werden. Der Vergleich von gemessenen Untersuchungsdaten mit archivierten Patientendaten und beispielhaften Referenzdaten ermöglicht eine Langzeitbeobachtung eines Patienten und somit die Feststellung gradueller und langsamer Veränderungen seiner Herzfunktionen.

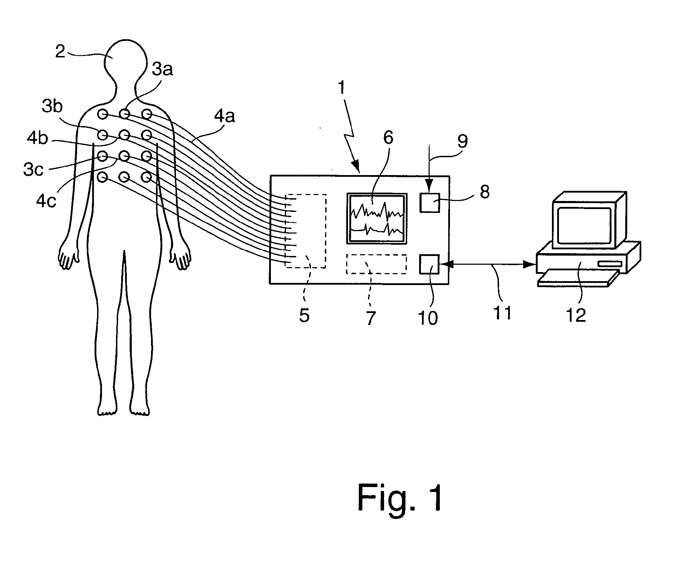

Weitere Vorteile und Merkmale der Erfindung ergeben sich aus der Beschreibung und der Figur der Zeichnung. Ebenso können die vorstehend genannten und die noch weiter aufgeführten Merkmale erfindungsgemäß jeweils einzeln oder in beliebigen Kombinationen miteinander verwendet werden. Das erfindungsgemäße Gerät ist in einem Ausführungsbeispiel in der Figur gezeigt. Die in der Figur gezeigten Merkmale sind rein schematisch und nicht maßstäblich zu verstehen.

Die Figur zeigt am Körper eines Patienten befestigte Elektroden zur Aufnahme kardiologischer Untersuchungsdaten durch ein erfindungsgemäßes Gerät und eine an das Gerät anschließbare Datenverarbeitungsanlage.

In der Figur ist ein Gerät 1 gezeigt, welches zur Aufnahme kardiologischer Untersuchungsdaten an einem Patienten 2 befestigt ist. Am Körper des Patienten 2 sind im Brustbereich insgesamt 12 Elektroden 3a, 3b, 3c, ... über einen Saugmechanismus fixiert. Die Elektroden 3a, 3b, 3c, ... sind jeweils über ein Kabel 4a, 4b, 4c, ... an eine Signalaufnahmeeinheit 5, welche im Gehäuse des Geräts 1 vorgesehen ist, angeschlossen. An den mit Bezugszeichen versehenen Elektroden 3a, 3b und 3c ist jeweils eine Schallaufnahmevorrichtung in Form eines Mikrophons angebracht. Die Mikrophone sind dabei derart in die Elektroden 3a, 3b und 3c integriert, dass der Herzschall des Patienten 2 weitgehend ohne äußere Störgeräusche aufgenommen werden kann. Die mittels der Elektroden 3a, 3b, 3c ... und der integrierten Schallaufnahmevorrichtungen erfassten akustischen und elektrischen Signale werden in einer Anzeigevorrichtung 6 angezeigt und in einem Datenspeicher 7 des Geräts 1 abgespeichert. Die Anzeigevorrichtung 6 ist ein Display, auf dem eine Herzstromkurve und ein Herzschallbild entsprechend der gemessenen Untersuchungsdaten dargestellt werden.

Das Gerät 1 weist weiter eine Eingabevorrichtung 8 auf, in die beispielsweise der Blutdruckwert des Patienten 2, wie mit Pfeil 9 angedeutet, eingegeben werden kann. Über eine Schnittstelle 10 kann eine mit Doppelpfeil 11 angedeutete Datenverbindung zwischen dem Gerät 1 und einer Datenverarbeitungsanlage 12, hier einem Computer, hergestellt werden. Die mit Hilfe des Geräts 1 am Körper des Patienten 2 abgenommenen Untersuchungsdaten können in der Datenverarbeitungsanlage 12 detailliert ausgewertet werden. Die Auswertung kann gleichzeitig mit, jedoch auch nach Aufnahme von Untersuchungsdaten am Patienten 2 durchgeführt werden und darüber hinaus an einem anderen Ort erfolgen.

Ein Gerät 1 zur Aufnahme kardiologischer Untersuchungsdaten an einem Patienten 2 weist mehrere Elektroden 3a, 3b, ... und mehrere in die Elektroden integrierte und/oder an diesen befestigte Schallaufnahmevorrichtungen auf, die am Körper des Patienten 2 befestigbar und mit einer Signalaufnahmeeinheit 5 verbunden sind. Mit Hilfe des Geräts 1 werden zeitgleich elektrische und akustische Signale vom Körper des Patienten 2 erfasst. Die gemessenen Untersuchungsdaten können sowohl mit Hilfe einer Anzeigevorrichtung 6 als Elektro- bzw. Phonokardiogramm dargestellt, als auch in einer Datenverarbeitungsanlage 12 zusätzlich gemeinsam ausgewertet und als Interpretation visuell angezeigt oder ausgedruckt werden.

Dies bedeutet, dass nicht nur ein EKG und ein Phonokardiogramm im bisherigen Sinn erstellt werden, sondern phonokardographisch detektierte Geräusche mit den Geräuschmomenten bekannter Herzklappenveränderungen verglichen werden.

Aus der Summe der so ermittelten Daten (EKG, Phonokardiogramm und gegebenenfalls manuell eingegebener weiterer Daten wie z. B. Blutdruck) wird dem Untersuchenden eine Gesamtinterpretation angeboten, deren Aussagewert den der bisherigen EKG- und Phonokardiographie-Diagnostik weit übertrifft und kein kardiologisches Expertenwissen des Untersuchenden erfordert.

Zielsetzung ist es - simultan zur EKG-Aufzeichnung - durch mehrere, in die EKG-Elektroden integrierte oder an diese adaptierte, Mikrophone, Herzgeräusche zu detektieren und diese zu einer Art dreidimensionalem Geräuschbild zusammenzufassen. Dieses wird mit in gleicher Weise erfassten Geräuschbildern bekannter Herzklappenveränderungen bekannten Ausmaßes rechnergestützt verglichen. Die daraus resultierende Interpretation kann anschließend ausgedruckt werden.

## Patentansprüche

1. Gerät (1) zur Aufnahme kardiologischer Untersuchungsdaten an einem Patienten (2), umfassend mehrere am Körper des Patienten (2) befestigbare Elektroden (3a, 3b, ...) und eine Signalaufnahmeeinheit (5), die mit den einzelnen Elektroden (3a, 3b, ...) verbunden ist und die von den Elektroden (3a, 3b, ...) am Körper des Patienten (2) erfassten Signale als Untersuchungsdaten aufnimmt,
**dadurch gekennzeichnet, dass**
das Gerät (1) zumindest eine mit der Signalaufnahmeeinheit (5) verbundene, an den Elektroden (3a, 3b, ...) befestigbare oder in diese integrierte Schallaufnahmevorrichtung aufweist.

2. Gerät nach Anspruch 1, **dadurch gekennzeichnet, dass** zumindest eine Schallaufnahmevorrichtung Mittel zum Anschluss an eine Elektrode (3a, 3b, ...) und/oder an die Signalaufnahmeeinheit (5), insbesondere einen Anschlussadapter, aufweist.

3. Gerät nach einem der vorhergehenden Ansprüche, **dadurch gekennzeichnet, dass** das Gerät (1) eine visuelle und/oder akustische Anzeigevorrichtung (6) zur Anzeige erfasster Untersuchungsdaten aufweist.

4. Gerät nach einem der vorhergehenden Ansprüche, **dadurch gekennzeichnet, dass** das Gerät (1) eine Schnittstelle (10) zur Ausbildung einer Datenverbindung mit einer Datenverarbeitungsanlage (12) aufweist.

5. Gerät nach einem der vorhergehenden Ansprüche, **dadurch gekennzeichnet, dass** das Gerät (1) einen Datenspeicher (7) zum Speichern der erfassten Untersuchungsdaten aufweist.

6. Gerät nach einem der vorhergehenden Ansprüche, **dadurch gekennzeichnet, dass** das Gerät (1) eine Eingabevorrichtung (8) zur Eingabe weiterer Untersuchungsdaten aufweist.

7. Gerät nach einem der vorhergehenden Ansprüche, **dadurch gekennzeichnet, dass** das Gerät (1) als Drei-, Sechs- oder Zwölfkanalgerät ausgebildet ist.

8. Verfahren zur Durchführung einer kardiologischen Untersuchung an einem Patienten unter Verwendung eines Geräts (1) nach einem der vorhergehenden Ansprüche, mit folgendem Verfahrensablauf:
(a) Befestigung der Elektroden (3a, 3b, ...) und der mindestens einen Schallaufnahmevorrichtung am Körper des Patienten (2);
(b) Erfassung von elektrischen und akustischen Signalen vom Körper des Patienten (2); und
(c) Abnahme der Elektroden (3a, 3b, ...) und der zumindest einen Schallaufnahmevorrichtung vom Patienten (2).

9. Verfahren nach Anspruch 8, **gekennzeichnet durch** die zusätzlichen Verfahrensschritte:
(d) Anschluss des Geräts (1) an eine Datenverarbeitungsanlage (12); und
(e) Auswertung der aufgenommenen Untersuchungsdaten **durch** die Datenverarbeitungsanlage (12),
wobei alternativ Verfahrensschritt (d) vor und Verfahrensschritt (e) gleichzeitig mit Verfahrensschritt (b) ausführbar ist.

10. Verfahren nach Anspruch 9, **dadurch gekennzeichnet, dass** die Untersuchungsdaten mit in der Datenverarbeitungsanlage (12) gespeicherten Referenzdaten verglichen werden und ein Untersuchungsergebnis ausgegeben wird.

11. Verfahren zum Betrieb eines Geräts (1) nach einem der Ansprüche 1 bis 7, mit folgenden Verfahrensschritten:
(A) Anschluss des Geräts (1) an eine Datenverarbeitungsanlage (12); und
(B) Auswertung der Untersuchungsdaten durch die Datenverarbeitungsanlage (12).

12. Verfahren nach Anspruch 11, **dadurch gekennzeichnet, dass** vor Verfahrensschritt (A) mithilfe des Geräts (1) Untersuchungsdaten aufgenommen und/oder Untersuchungsdaten in das Gerät (1) eingegeben werden.

13. Verfahren nach Anspruch 11 oder 12, **dadurch gekennzeichnet, dass** die Untersuchungsdaten mit in der Datenverarbeitungsanlage (12) gespeicherten Referenzdaten verglichen werden und ein Untersuchungsergebnis ausgegeben wird.
